# EUROPEAN PATENT SPECIFICATION

(11) **EP 1 928 368 B1**
(45) Date of publication and mention of the grant of the patent: **21.12.2011**
(21) Application number: 06752080.9
(22) Date of filing: 04.05.2006
(51) Int. Cl.: A61F 2/86

(54) **HYBRID BIFURCATED STENT**
GEGABELTER HYBRIDSTENT
STENT BIFURQUÉ HYBRIDE

(30) Priority: 30.09.2005 US 240313
(43) Date of publication of application: 11.06.2008
(73) Proprietor: Boston Scientific Limited, Christ Church (BB)
(72) Inventor: EHR, Timothy, Elk River, MN 55330 (US); MERDAN, Kenneth, Greenfield, MN 55357-8716 (US); RASSAT, Jay, Buffalo, MN 55313 (US); SUTERMEISTER, Derek, Eden Prairie, MN 55436 (US)
(74) Representative: Hauck Patent- und Rechtsanwälte
(86) International application number: PCT/US2006/016794
(87) International publication number: WO 2007/040630

(56) References cited:
- EP-A- 0 904 745
- WO-A-02/30329
- DE-A1- 19 818 601
- US-A1- 2002 183 763

## Description

### BACKGROUND OF THE INVENTION

### Field of the Invention

In some embodiments this invention relates to implantable medical devices, their manufacture, and methods of use. Some embodiments are directed to delivery systems, such as catheter systems of all types, which are utilized in the delivery of such devices.

### Description of the Related Art

A stent is a medical device introduced to a body lumen and is well known in the art. Typically, a stent is implanted in a blood vessel at the site of a stenosis or aneurysm endoluminally, i.e. by so-called "minimally invasive techniques" in which the stent in a radially reduced configuration, optionally restrained in a radially compressed configuration by a sheath and/[omicron]r catheter, is delivered by a stent delivery system or "introducer" to the site where it is required. The introducer may enter the body from an access location outside the body, such as through the patient's skin, or by a "cut down" technique in which the entry blood vessel is exposed by minor surgical means.

Stents, grafts, stent-grafts, vena cava filters, expandable frameworks, and similar implantable medical devices, collectively referred to hereinafter as stents, are radially expandable endoprostheses which are typically intravascular implants capable of being implanted transluminally and enlarged radially after being introduced percutaneously. Stents may be implanted in a variety of body lumens or vessels such as within the vascular system, urinary tracts, bile ducts, fallopian tubes, coronary vessels, secondary vessels, etc. Stents may be used to reinforce body vessels and to prevent restenosis following angioplasty in the vascular system. They may be self-expanding, expanded by an internal radial force, such as when mounted on a balloon, or a combination of self-expanding and balloon expandable (hybrid expandable).

Stents may be created by methods including cutting or etching a design from a tubular stock, from a flat sheet which is cut or etched and which is subsequently rolled or from one or more interwoven wires or braids.

Within the vasculature it is not uncommon for stenoses to form at a vessel bifurcation. A bifurcation is an area of the vasculature or other portion of the body where a first (or parent) vessel is bifurcated into two or more branch vessels. Where a stenotic lesion or lesions form at such a bifurcation, the lesion(s) can affect only one of the vessels (i.e., either of the branch vessels or the parent vessel) two of the vessels, or all three vessels. Many prior art stents however are not wholly satisfactory for use where the site of desired application of the stent is juxtaposed or extends across a bifurcation in an artery or vein such, for example, as the bifurcation in the mammalian aortic artery into the common iliac arteries.

WO 02/30329 A2 discloses a covered stent having a side branch. The branch portion of the stent extends from the intermediate portion of the main portion at an angle thereto and is in fluid communication with the main portion.

The problem of the invention is to provide an improved stent for vessel bifurcations. The problem is solved according to claim 1.

Without limiting the scope of the invention a brief summary of some of the claimed embodiments of the invention is set forth below. Additional details of the summarized embodiments of the invention and/or additional embodiments of the invention may be found in the Detailed Description of the Invention below.

### BRIEF SUMMARY OF THE INVENTION

In at least one embodiment, the invention is directed to a stent assembly having a first stent body and a second stent body. The first stent body defines a first lumen having a first longitudinal axis therethrough, and the second stent body defines a second lumen having a second longitudinal axis therethrough. An end of the second stent body is hingedly engaged to a portion of the first stent body, and the first lumen and the second lumen are in fluid communication with one another. The stent assembly has a undeployed and a deployed state. In the undeployed state the first longitudinal axis and the second longitudinal axis are substantially parallel to one another and the second lumen is positioned entirely within the first lumen. In the deployed state the second longitudinal axis forms an oblique angle with the first longitudinal axis. For the purpose of this application, the term "oblique" refers to an angle of between 1 and 180 degrees and explicitly includes angles of about 90 degrees. The first stent body is comprised of a plurality of interconnected first stent members, in at least one embodiment, adjacent first stent members define a plurality of openings through the first stent body in fluid communication with the first lumen.

The second stent body is comprised of a plurality of interconnected second stent members wherein adjacent second stent members define a plurality of openings through the second stent body. In at least one embodiment, the openings are in fluid communication with the second lumen. In at least one embodiment, the first stent members form a first stent configuration and the second stent members form a second stent configuration.

According to the invention, the first stent configuration and the second stent configuration have different expansion characteristics.

In at least one embodiment, the second stent body can be defined by an inner region and an outer region wherein the outer region may be a portion of the first stent body and the inner region may be engaged to a portion of the first stent body. In at least one embodiment, the first stent body is substantially tubular in shape, hi at least one embodiment, the tube has an oval cross-section.

In at least one embodiment, in the undeployed shape the second stent body has a substantially ovoid cross-sectional shape. In some embodiments the unexpanded stent body has a substantially flattened cross-sectional shape, in at least one embodiment, in the deployed state the second stent body has a substantially round cross-sectional shape. Other possible shapes include hexagonal, triangular, square, rhombus, etc.

In at least one embodiment, each of the openings has an area. In at least one embodiment, at least one of the openings can be characterized as a side branch opening having an area greater than that of each of the other openings.

In at least one embodiment, the side branch opening is immediately adjacent to the end of the second stent body which is hingedly or otherwise flexibly engaged to a portion of the first stent body. In at least one embodiment, the side branch opening is in fluid communication with the first lumen and the second lumen.

In at least one embodiment, at least a portion of at least one of the first stent body and the second stent body is substantially self-expandable from the undeployed state to the deployed state.

In at least one embodiment, at least a portion of at least one of the first stent body and the second stent body is substantially expandable from the undeployed state to the deployed state by application of a radially outward acting force.

In at least one embodiment, the second stent body is defined by an inner region and an outer region such that in the undeployed state the inner region and the outer region can be a portion of the first stent body and in the deployed state the second stent body can have a substantially round cross-sectional shape.

In at least one embodiment, a bifurcated stent delivery system has a catheter shaft having a device receiving portion and a stent disposed about the device receiving portion. In at least one embodiment, the stent has a stent body and a primary longitudinal axis wherein a portion of the stent body is a flap hingedly attached to an attachment portion of the stent body. The stent has a deployed state and an undeployed state. In at least one embodiment, in the undeployed state the flap extends in a direction substantially parallel to the longitudinal axis. In at least one embodiment, in the deployed state the flap expands into a tubular shape and has a secondary longitudinal axis which extends in a direction oblique to the longitudinal axis. In at least one embodiment, the flap is constructed of alternating first stent members and second stent members, in the undeployed state the first and second stent members substantially the same distance from the primary longitudinal axis, in the deployed state the first stent members and the second stent members on opposite sides of a plane passing through the secondary longitudinal axis.

In at least one embodiment, the flap is constructed of alternating first stent members and second stent members, in the undeployed state the first and second stent members substantially the same distance from the primary longitudinal axis, in the deployed state the first stent members and the second stent members on opposite sides of a plane passing through the secondary longitudinal axis.

In at least one embodiment, the flap is an integral part of the stent body.

In at least one embodiment, the flap is constructed of a different material than the rest of the stent body.

In at least one embodiment a stent system includes a stent device, a catheter, and a push and/or pull device(s). The stent device has an expanded state and an unexpanded state and comprises multiple wires loosely attached to one another. The stent device is self-expanding. The stent device is in the unexpanded state when disposed within the catheter. The stent device is in the expanded state when unrestrained outside the catheter. When disposed within the catheter the wires are in direct contact with the walls of the inner lumen of the catheter. The push and/or pull device can be removably attached to at least one wire. Distal movement of the push device forces the stent out the end of the catheter and into the expanded state. Proximal movement of the pull device forces the stent into the catheter and into the unexpanded state.

In at least one embodiment, the push device and the pull device are the same.

In at least one embodiment, the wires are attached by the geometry of each wire, by rings, by swaging, by swaged connectors, by welds, by adhesive bonding, by mechanical attachments other than swaging (e.g. tolerance fits, press fit, etc.) and/or cuffs.

In at least one embodiment, the stent device is capable of moving from the expanded state to the unexpanded state multiple times while within a body lumen.

In at least one embodiment, the wires are pre-formed over a mandrel, form, mold, etc. In at least one embodiment, the wires are customly formed to match the treatment site of the body lumen.

In at least one embodiment, the stent device is substantially longer in the unexpanded state than when in the expanded state.

In at least one embodiment, the stent device is up to 200mm long.

In at least one embodiment, the stent system includes a sheath. The sheath at least partially disposed within the catheter and in the unexpanded state the stent device disposed within the sheath.

In at least one embodiment, the sheath can be proximally pulled back to release the stent device into a body lumen for expansion.

These and other embodiments which characterize the invention are pointed out with particularity in the claims annexed hereto and forming a part hereof. However, for further understanding of the invention, its advantages and objectives obtained by its use, reference should be made to the drawings which form a further part hereof and the accompanying descriptive matter, in which there is illustrated and described embodiments of the invention.

### BRIEF DESCRIPTION OF THE SEVERAL VIEWS OF THE DRAWING(S).

A detailed description of the invention is hereafter described with specific reference being made to the drawings.
FIG. 1 is a side view of a flat representation of an embodied stent.
FIG. 2 is a cross-sectional view of an inventive stent.
FIG. 3 is a cross-sectional side view of an inventive flap.
FIG. 4 is a side view of an embodied stent.
FIG. 5 is a perspective top view of an embodied stent.
FIG. 6 is a perspective top view of an embodied flap.
FIG. 6a is a cross-sectional view of the flap lumen.
FIG. 7 is a side view of stent with an inflation balloon disposed within the flap lumen.
FIG. 8 is a top view of an embodied stent.
FIG. 9 is a side view of stent with an inflation balloon disposed within the flap lumen.
FIG. 10 is a cross-sectional side view of a stent delivery apparatus.
FIG. 11 is a cross-sectional side view of a stent delivery apparatus.
FIG. 12 is a cross-sectional side view of a stent delivery apparatus.
FIG. 13 is a cross-sectional side view of a stent delivery apparatus
FIG. 14 is a cross-sectional side view of a stent delivery apparatus

### DETAILED DESCRIPTION OF THE INVENTION

While this invention may be embodied in many different forms, there are described in detail herein specific preferred embodiments of the invention. This description is an exemplification of the principles of the invention and is not intended to limit the invention to the particular embodiments illustrated.

For the purposes of this disclosure, like reference numerals in the figures shall refer to like features unless otherwise indicated.

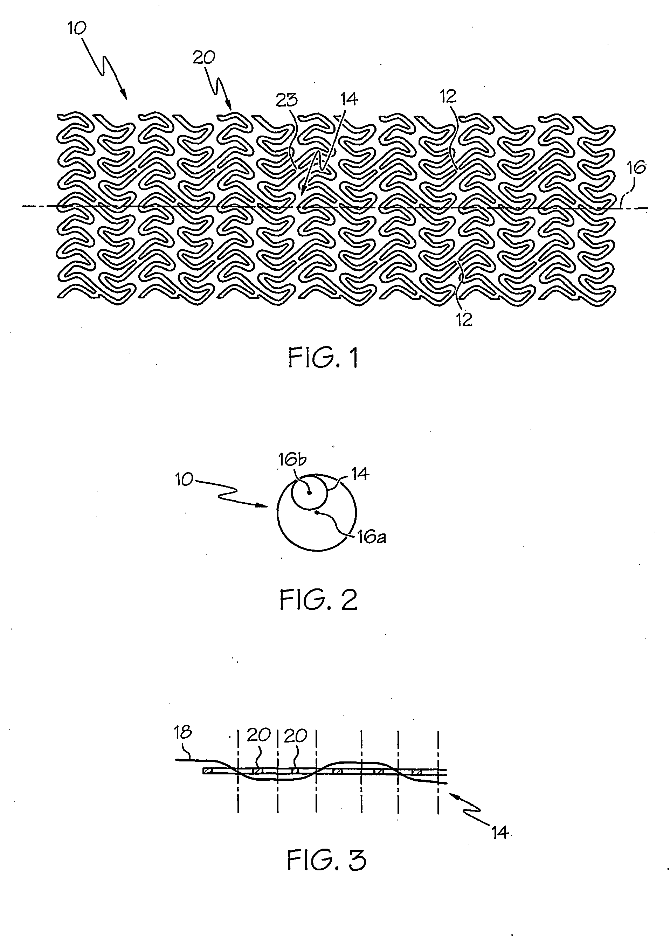

In Fig. 1 a flat representation of an embodied stent 10 having longitudinal axis 16 is shown. The stent 10 has two hinge points 12. The hinge points 12 allow a portion or flap 14 of the stent 10 to pivot out from the remainder of the stent 10. In the undeployed the flap 14 is disposed about the longitudinal axis at substantially the same radial distance as the rest of the stent 10. In some embodiments as illustrated in Fig. 2, the flap 14 may be a second stent body which includes in the undeployed state an independent secondary lumen. The second stent body 14 resides within the primary lumen of the stent 10. In this embodiment, the primary and the secondary lumens have separate and parallel longitudinal axes 16a and 16b.

In some embodiments, as shown in Fig. 3, a secondary guidewire 18 is woven through the struts 20 of the flap 14 in an alternating sequence. In some instances the guidewire 18 is woven between adjacent struts 20 of the flap 14 and in other embodiments, the guidewire alternates between every two or more struts. The secondary guidewire 18 can serve multiple purposes. The guidewire 18 can guide an expansion balloon as it is advanced between alternating struts or alternating groups of struts 20 such that the flap 14 can be expanded and/or deployed by the expansion of the expansion balloon.

Additionally, the guidewire 18 can be woven through the flap 14 as well as a portion of the primary stent body and thus prevent the flap 14 from pivoting outward or opening prior to deployment. The secondary guidewire 18 can then be partially retracted such that the guidewire 18 is no longer woven into the primary stent body as well as the flap 14 to allow the flap 14 to pivot about the hinge 12 and be deployed.

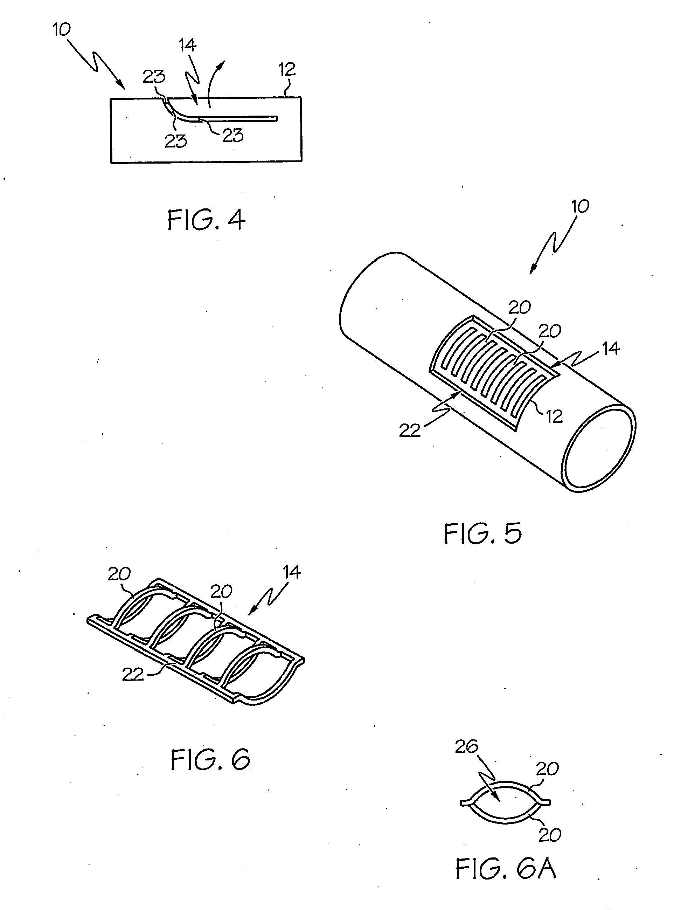

In Fig. 4 another representative stent 10 having a flap 14 is shown. The stent 10 can be of any of a variety of stents including coil stents, segmented stents, block stents, etc. As shown the flap 14 pivots about hinge points 12. In some embodiments, frangible members 23 can be used to assist in holding the flap in place during delivery. In embodiments having frangible members, the frangible members 23 can be designed to dissolve within a specific period of time or to be broken with the application of radial outward force within the stent 10. The frangible members can be disposed opposite the hinged side of the flap 14. The frangible members can also be disposed along the sides of the flap 14. The frangible members may be struts which are made of a different material than the rest of the stent and/or are made smaller/narrower than other portions of the stent. The different material or size difference creates a weaker portion that breaks upon expansion of the stent 10.

A perspective top view of an embodied stent 10 is shown in Fig. 5 wherein the flap 14 is illustrated as having strut members 20 substantially parallel to one another and connected to one another by frame 22. The flap 14 can be of a separate and distinct pattern from the primary stent portion. This can enable the flap to have different expansion characteristics than the primary stent body. The stent 10 can be made of a single piece of tubing such that before deployment the flap is incorporated into the wall of the primary stent body.

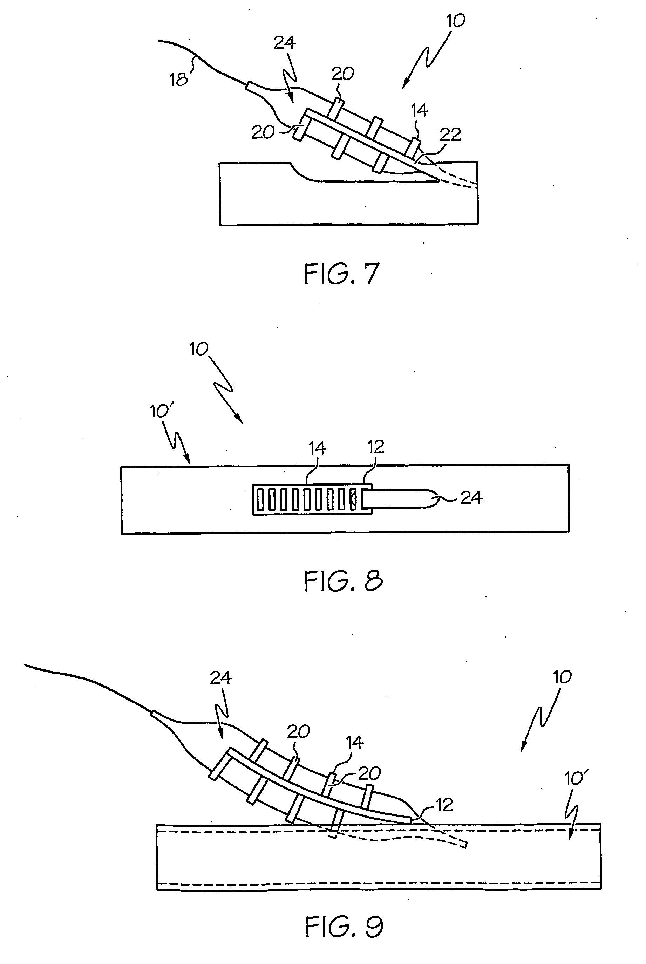

As shown in Fig. 6 and 6a, in the expanded state or the deployed state the struts 20 of the flap 14 can expand such that adjacent struts bow in opposite directions within the frame 22. In some embodiments, the flap 14 in an expanded or deployed state might have an ovoid shape. Other possible shapes include hexagonal, triangular, square, rhombus, etc. In some embodiments adjacent groups of struts bow in opposite directions upon expansion. This expansion can be self-expansion or can also be through the use of an expansion balloon as shown in Fig. 7.

In Fig. 7 the flap is expanded by expansion of an inflation balloon 24 which is advanced about the secondary guidewire 18. The guidewire 18 can be woven between adjacent struts 20 such that expansion of the advanced balloon 24 forces adjacent struts to bow in opposing directions thereby creating a secondary lumen 26 similar to that shown in Fig. 6a.

Both the primary and secondary lumen can be inflated by the same balloon. Two different balloons can also be used in some embodiments. In some embodiments a smaller more specialized balloon (e.g. <1.0 DIA) is used in expanding the secondary lumen of flap 14 and/or is used to initially open the secondary lumen so another inflation balloon can be inserted. Multiple balloon catheters such as those disclosed in U.S. patent 6,780,174 to Mauch are known in the art.

In some embodiments as shown in Figs. 8 and 9, the flap 14 is not integral with the primary stent body portion 10'. In some embodiments, the flap 14 is attached to the outside of the primary stent body 10' at hinge point 12. In this embodiment, less of the primary stent body is repositioned into a secondary lumen thereby maximizing the scaffolding within the primary lumen.

In at least one embodiment, a strut is removed from the primary stent body 10' such that an inflation balloon 24 can pass through the wall of primary stent body 10' and advance between the struts 20 of flap 14. In some embodiments, the flap 14 and the primary stent body 10' can be constructed of different materials such that varying performance criteria can be attained. The flap can also be constructed of a NiTi alloy and self-expanded upon withdrawal of a sheath.

In some embodiments, an attached flap 14 rather than an integral flap 14 allows the flap to be longer as structural support is not being repositioned from the primary stent body 10' to the secondary lumen.

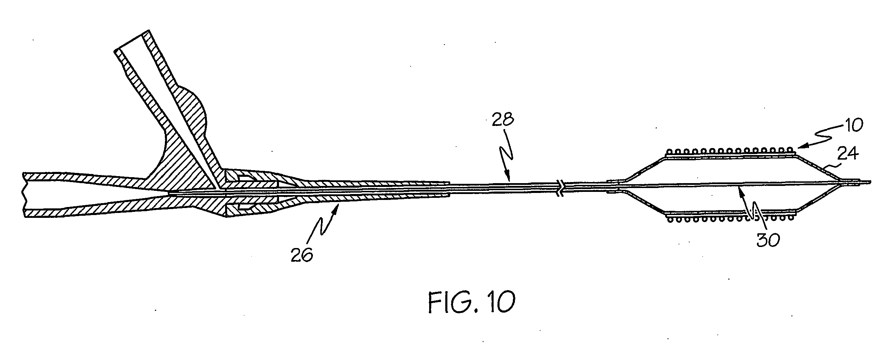

As shown in Fig. 10, the various embodied stents 10 can be a part of a stent delivery system 26 having a catheter shaft 28 with stent receiving region 30. The stent 10 can be disposed about a balloon. 24. While only a single balloon is shown here, multiple balloons can be used. One balloon can be used to expand the primary body portion of stent 10 while another balloon is used for entering the secondary lumen and expanding the flap.

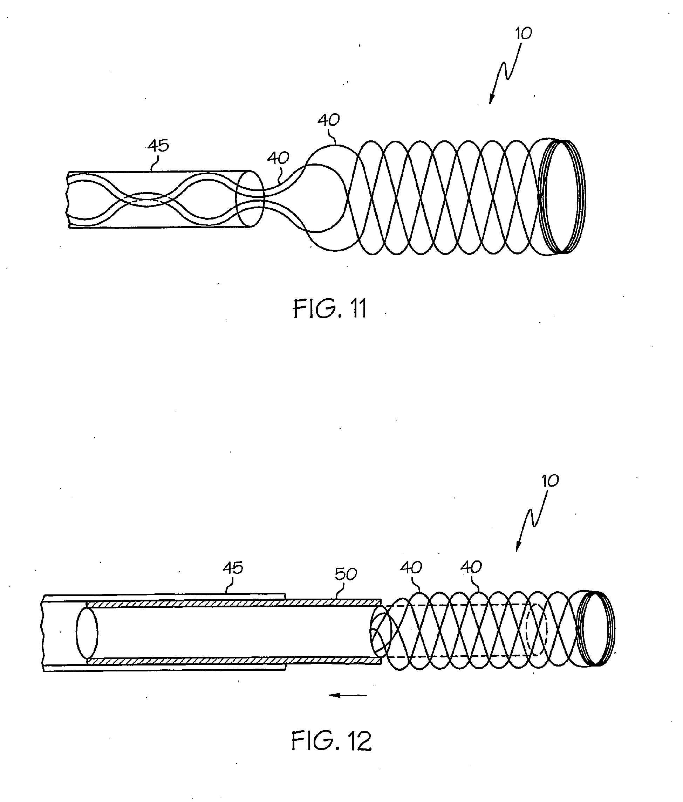

In some embodiments, the stent device is formed from a series of wires 40 that may or may not be joined together. The wires are formed in a geometric pattern to form a self expanding stent. The wires comprising the stent device can be preformed outside of a delivery system on a mandrel, form, mold, etc. The wires 40 can be formed around a mold or fixture either individually or separately. The wires 40 can be stressed or formed to a non-plastically deformed state that will provide the desired radial strength, profile, diameter, scaffolding, etc. The wires 40 are can be arranged together such that they will not detach from one another. The wires can then be pulled into a low profile delivery system 45 or catheter. The catheter 45 can then be inserted into the body to an affected area where the stent device 10 can be deployed by being pushed out of the delivery system 45 or, as shown in Fig. 12, by drawing back a sheath 50 and leaving the stent device 10 in place.

The stent devices 10 of Figs. 11-12 have several embodied advantages. One embodied advantage is that if the necessary target area is not attained on the first delivery attempt the stent device 10 can be retrieved by pulling the wires 40 of the stent device 10 back into the catheter 45.

In at least one embodiment, the stent device 10 can be extremely long (up to 200 mm); the device 10 can be constructed without welds which addresses fatigue issues within welds or other connecting points in extremely long stents. In one embodiment the stent device 10 is constructed without welds, but rather by changes in direction of the geometry of the wires 40. In some embodiments full or partial loops keep the wires 40 from detaching from one another while at the same time maintaining individual movement of the wires 40. Thus, less stress is placed on junction areas of the device. In some embodiments the wires could be formed or joined together with rings, swaged connectors or cuffs.

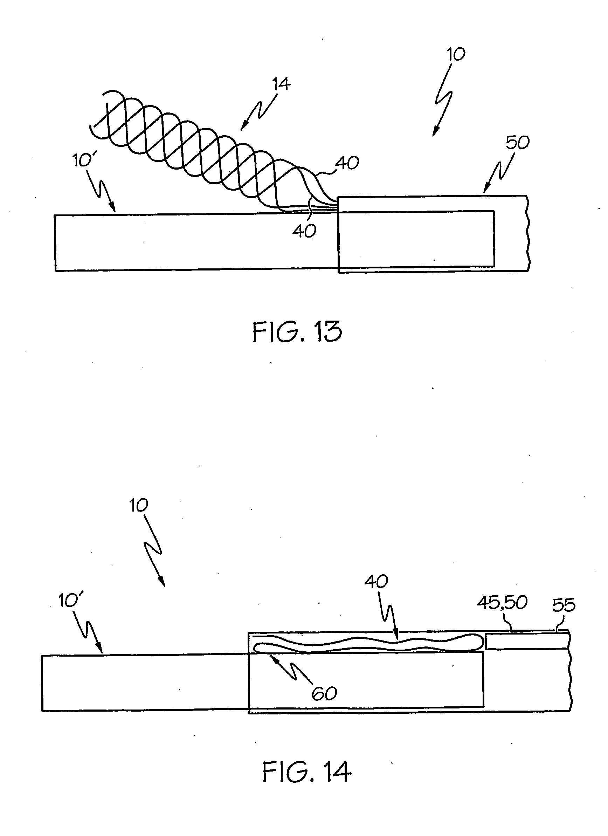

In some embodiments as shown in Fig. 13, the flap 14 is formed from one or more wires 40 similar to those of Figs. 11 and 12 which may or may not be joined together. In some embodiments, the wires are welded together or joined together with rings, swaged connectors, or cuffs. In some embodiments, the flap 14 is not integral to the stent body portion 10'. The flap 14 may be attached to the stent body portion 10' outside the body or within the body, even in the area of the treatment site.

In some embodiments, the flap 14 may be integral with the primary stent body portion 10'. The flap 14 may be attached to the outside of the primary stent body 10', in some cases at a hinge point as the flap 14 of Fig. 9 is attached. The flap may be constructed of the same or of a different material than that of the primary stent body 10'.

Whether integral or not to the stent body portion 10', prior to delivery the wires 40 of flap 14 maybe restrained within a catheter. In some embodiments, the wires 40 are retained within a sheath 50 and then delivered upon retraction of the sheath as shown in Fig. 13. The sheath 50 and stent device of Fig. 13 may be disposed within a catheter or other delivery assembly.

In Fig. 14 one or more wires 40 of flap 14 are contained within the catheter 45 or the sheath 50. As shown, the one or more wires 40 of flap 14 may be pushed distally in order to be delivered. In at least one embodiment, an optional push rod 55 can assist in pushing the wires 40 forward for delivery. A push rod 55 can be particularly useful in distally pushing the wires 40 when the wires are attached to the primary stent body 10' at connection 60.

In at least one embodiment, a sheath 50 can be an optional feature and can extend distally beyond the end of the catheter. In at least one embodiment, the flap 14 may be delivered by both pushing the at least one wire 40 forward using the wire(s) or the push rod 55 described above and by retracting the sheath 50.

In at least one embodiment, a strut is removed from the primary stent body 10' such that an inflation balloon 24 can pass through the wall of primary stent body 10' and advance between the struts 20 of flap 14. In some embodiments, the flap 14 and the primary stent body 10' can be constructed of different materials such that varying performance criteria can be attained. The flap can also be constructed of a NiTi alloy and self-expanded upon withdrawal of a sheath.

In some embodiments, an attached flap 14 rather than an integral flap 14 allows the flap to be longer as structural support is not being repositioned from the primary stent body 10'to the secondary lumen.

In some embodiments the wires of the stent device 10 may comprise filament tubes or filament rods. The wires, filament tubes, and/or filament rods may be constructed ofNitinol or other shape memory metal, titanium, stainless steel, Elgiloy, NP35N, Hastelloy, or other alloyed metals. Shape memory polymers such as cross linked polyurethanes, polynorbomene, poly dimethacrylate, and biodegradable shape memory polymers such as oligo(ε-caprolactone)diol.

In some embodiments, the wires could be pre-stressed to a plastic state and formed inside the body. This can allow the system to be built inside of the legion or affected area.

In some embodiments the stent, the delivery system or other portion of the assembly may include one or more areas, bands, coatings, members, etc. that is (are) detectable by imaging modalities such as X-Ray, MRI, ultrasound, etc. In some embodiments at least a portion of the stent and/or adjacent assembly is at least partially radiopaque.

In some embodiments the at least a portion of the stent is configured to include one or more mechanisms for the delivery of a therapeutic agent. Often the agent will be in the form of a coating or other layer (or layers) of material placed on a surface region of the stent, which is adapted to be released at the site of the stent's implantation or areas adjacent thereto.

A therapeutic agent maybe a drug or other pharmaceutical product such as non-genetic agents, genetic agents, cellular material, etc. Some examples of suitable non-genetic therapeutic agents include but are not limited to: anti-thrombogenic agents such as heparin, heparin derivatives, vascular cell growth promoters, growth factor inhibitors, Paclitaxel, etc. Where an agent includes a genetic therapeutic agent, such a genetic agent may include but is not limited to: DNA, RNA and their respective derivatives and/or components; hedgehog proteins, etc. Where a therapeutic agent includes cellular material, the cellular material may include but is not limited to: cells of human origin and/or non-human origin as well as their respective components and/or derivatives thereof. Where the therapeutic agent includes a polymer agent, the polymer agent maybe a polystyrene-polyisobutylene-polystyrene triblock copolymer (SEBS), polyethylene oxide, silicone rubber and/or any other suitable substrate.

## Claims

1. A stent assembly (10) having an undeployed state and a deployed state, the assembly comprising:
a first stent body (10'), the first stent body defining a first lumen having a first longitudinal axis (16a) therethrough, the first stent body (10') is comprised of a plurality of interconnected first stent members, adjacent first stent members defining a plurality of openings through the first stent body in fluid communication with the first lumen, the first stent members forming a first stent configuration; and
a second stent body (14), the second stent body (14) defining a second lumen having a second longitudinal axis (16b) therethrough, an end of the second stent body (14) hingedly engaged to a portion of the first stent body (10'), the first lumen and the second lumen in fluid communication with one another, in the undeployed state the first longitudinal axis (16a) and the second longitudinal axis (16b) being substantially parallel to one another and the second lumen positioned entirely within the first lumen, in the deployed state the second longitudinal axis forming an oblique angle with the first longitudinal axis (16a), wherein the second stent body is comprised of a plurality of interconnected second stent members, adjacent second stent members defining a plurality of openings through the body in fluid communication with the second lumen, the second stent members forming a second stent configuration, **characterized in that** the first stent configuration and the second stent configuration have different expansion characteristics.

2. The assembly of claim 1 wherein the second stent body is defined by an inner region and an outer region, the outer region being a portion of the first stent body, the inner region engaged to the portion of the first stent body.

3. The assembly of claim 1 wherein the first stent body is substantially tubular in shape.

4. The assembly of claim 1 wherein in the undeployed shape the second stent body has a substantially ovoid cross-sectional shape and in the deployed state the second stent body has a substantially round cross-sectional shape.

5. The assembly of claim 1 wherein each of the openings has an area, at least one of the openings characterized as a side branch opening having an area greater than that of each of the openings.

6. The assembly of claim 5 wherein the side branch opening is immediately adjacent to the end of the second stent body hingedly engaged to the portion of the first stent body.

7. The assembly of claim 6 wherein the side branch opening is in fluid communication with the first lumen and the second lumen.

8. The assembly of claim 1 wherein at least a portion of at least one of the first stent body and the second stent body is substantially self-expandable from the undeployed state to the deployed state.

9. The assembly of claim 1 wherein at least a portion of at least one of the first stent body and the second stent body is substantially expandable from the undeployed state to the deployed state by application of a radially outward acting force.

10. The assembly of claim 1 wherein the second stent body is defined by an inner region and an outer region, in the undeployed state the inner region and the outer region being a portion of the first stent body, in the deployed state the second stent body has a substantially round cross-sectional shape.

## Patentansprüche

1. Stentbaugruppe (10) mit einem nicht entfalteten Zustand und einem entfalteten Zustand, wobei die Baugruppe umfasst:
einen ersten Stentkörper (10'), wobei der erste Stentkörper ein erstes Lumen mit einer ersten Längsachse (16a) durch es hindurch definiert, der erste Stentkörper (10') aus einer Vielzahl von untereinander verbundenen ersten Stentelementen besteht, benachbarte erste Stentelemente eine Vielzahl von Öffnungen durch den ersten Stentkörper hindurch in Fluidkommunikation mit dem ersten Lumen definieren und die ersten Stentelemente eine erste Stentkonfiguration bilden; und
einen zweiten Stentkörper (14), wobei der zweite Stentkörper (14) ein zweites Lumen mit einer zweiten Längsachse (16b) durch es hindurch definiert, ein Ende des zweiten Stentkörpers (14) schwenkbar mit einem Abschnitt des ersten Stentkörpers (10') in Eingriff ist, das erste und das zweite Lumen in Fluidkommunikation miteinander sind, in dem nicht entfalteten Zustand die erste Längsachse (16a) und die zweite Längsachse (16b) im wesentlichen parallel zueinander sind und das zweite Lumen vollständig innerhalb des ersten Lumens positioniert ist, in dem nicht entfalteten Zustand die zweite Längsachse mit der ersten Längsachse (16a) einen stumpfen Winkel erzeugt, wobei der zweite Stentkörper aus einer Vielzahl von untereinander verbunden zweiten Stentelementen besteht, benachbarte zweite Stentelemente eine Vielzahl von Öffnungen durch den Körper hindurch in Fluidkommunikation mit dem zweiten Lumen definieren und die zweiten Stentelemente eine zweite Stentkonfiguration erzeugen, **dadurch gekennzeichnet, dass** die erste Stentkonfiguration und die zweite Stentkonfiguration unterschiedliche Expansionsmerkmale haben.

2. Baugruppe gemäß Anspruch 1, wobei der zweite Stentkörper durch ein inneres Gebiet und ein äußeres Gebiet definiert ist, das äußere Gebiet ein Abschnitt des ersten Stentkörpers ist und das innere Gebiet mit dem Abschnitt des ersten Stentkörpers in Eingriff ist.

3. Baugruppe gemäß Anspruch 1, wobei der erste Stentkörper von im wesentlichen röhrenförmiger Form ist.

4. Baugruppe gemäß Anspruch 1, wobei der zweite Stentkörper in dem nicht entfalteten Zustand eine im wesentlichen ovale Querschnittsform hat, und der zweite Stentkörper in dem entfalteten Zustand eine im wesentlichen runde Querschnittsform hat.

5. Baugruppe gemäß Anspruch 1, wobei jede der Öffnungen eine Fläche hat und mindestens eine der Öffnungen, die als eine Seitenzweigöffnung gekennzeichnet ist, eine größere Fläche als diejenige von allen anderen Öffnungen hat.

6. Baugruppe gemäß Anspruch 5, wobei die Seitenzweigöffnung unmittelbar angrenzend an das schwenkbar mit dem Abschnitt des ersten Stentkörpers in Eingriff befindliche Ende des zweiten Stentkörpers ist.

7. Baugruppe gemäß Anspruch 6, wobei die Seitenzweigöffnung in Fluidkommunikation mit dem ersten Lumen und dem zweiten Lumen ist.

8. Baugruppe gemäß Anspruch 1, wobei mindestens ein Abschnitt von mindesten einem aus dem ersten Stentkörper und dem zweiten Stentkörper im wesentlichen selbstexpandierbar aus dem nicht entfalteten Zustand zu dem entfalteten Zustand ist.

9. Baugruppe gemäß Anspruch 1, wobei mindestens ein Abschnitt von mindesten einem aus dem ersten Stentkörper und dem zweiten Stentkörper durch Aufbringen einer radial nach außen wirkenden Kraft im wesentlichen aus dem nicht entfalteten Zustand zu dem entfalteten Zustand expandierbar ist.

10. Baugruppe gemäß Anspruch 1, wobei der zweite Stentkörper durch ein inneres Gebiet und ein äußeres Gebiet definiert ist, in dem nicht entfalteten Zustand das innere Gebiet und das äußere Gebiet ein Abschnitt des ersten Stentkörpers sind und in dem entfalteten Zustand der zweite Stentkörper eine im wesentlichen runde Querschnittsform hat.

## Revendications

1. Ensemble de Stent (10) avec une condition non déployée et une condition déployée, l'ensemble comprenant:
un premier corps de Stent (10'), le premier corps de Stent définissant une première lumière avec un premier axe longitudinal (16a) à travers cela, le premier corps de Stent (10') consistant d'une pluralité de premiers éléments de Stent interconnectés, des premiers éléments de Stent adjacents définissant une pluralité d'ouvertures à travers le premier corps de Stent en communication fluidique avec la première lumière et les premiers éléments de Stent formant une première configuration de Stent; et
un deuxième corps de Stent (14), le deuxième corps de Stent (14) définissant une deuxième lumière avec un deuxième axe longitudinal (16b) à travers cela, une extrémité du deuxième corps de Stent (14) étant engagée de façon pivotante avec une portion du premier corps de Stent (10'), les lumières première et deuxième étant en communication fluidique l'une avec l'autre, dans la condition non déployée les axes longitudinaux premier (16a) et deuxième (16b) étant essentiellement parallèles l'un à l'autre et la deuxième lumière étant positionnée entièrement au-dedans de la première lumière, dans la condition non déployée le deuxième axe longitudinal formant un angle oblique avec le premier axe longitudinal (16a), le deuxième corps de Stent consistant d'une pluralité de deuxièmes éléments de Stent interconnectés, des deuxièmes éléments de Stent adjacents définissant une pluralité d'ouvertures à travers le corps en communication fluidique avec la deuxième lumière et les deuxièmes éléments de Stent formant une deuxième configuration de Stent, **caractérisé en ce que** la première configuration de Stent et la deuxième configuration de Stent ont des caractéristiques d'expansion différentes.

2. Ensemble selon la revendication 1, dans lequel le deuxième corps de Stent est défini par une région intérieure et une région extérieure, la région extérieure étant une portion du premier corps de Stent et la région intérieure étant engagée avec la portion du premier corps de Stent.

3. Ensemble selon la revendication 1, dans lequel le premier corps de Stent est d'une forme essentiellement tubulaire.

4. Ensemble selon la revendication 1, dans lequel dans la condition non déployée, le deuxième corps de Stent a une forme de la coupe transversale essentiellement ovale, et dans la condition déployée, le deuxième corps de Stent a une forme de la coupe transversale essentiellement circulaire.

5. Ensemble selon la revendication 1, dans lequel chacune des ouvertures a une aire et au moins une des ouvertures, caractérisée comme une ouverture de branche latérale, a une aire plus grande que celle de tous les autres ouvertures.

6. Ensemble selon la revendication 5, dans lequel l'ouverture de branche latérale est immédiatement adjacente à l'extrémité du deuxième corps de Stent engagée de façon pivotante à la portion du premier corps de Stent.

7. Ensemble selon la revendication 6, dans lequel l'ouverture de branche latérale est en communication fluidique avec la première lumière et la deuxième lumière.

8. Ensemble selon la revendication 1, dans lequel au moins une portion d'au moins un des corps de Stent premier et deuxième est essentiellement auto-dilatable à partir de la condition non déployée vers la condition déployée.

9. Ensemble selon la revendication 1, dans lequel au moins une portion d'au moins un des corps de Stent premier et deuxième est essentiellement dilatable à partir de la condition non déployée vers la condition déployée par l'application d'une force agissant radialement vers l'extérieur.

10. Ensemble selon la revendication 1, dans lequel le deuxième corps de Stent est défini par une région intérieure et une région extérieure, dans la condition non déployée, la région intérieure et la région extérieure étant une portion du premier corps de Stent et dans la condition déployée le deuxième corps de Stent ayant une forme de la coupe transversale essentiellement circulaire.
